# EUROPÄISCHE PATENTSCHRIFT

(11) **EP 0 783 484 B1**
(45) Veröffentlichungstag und Bekanntmachung des Hinweises auf die Patenterteilung: **02.06.1999**
(21) Anmeldenummer: 95932722.2
(22) Anmeldetag: 12.09.1995
(51) Int. Cl.: C07C 231/02, C07C 233/47

(54) **VERFAHREN ZUR HERSTELLUNG VON N-ACYLAMINOCARBONSÄUREN UND N-ACYLAMINOSULFONSÄUREN SOWIE DEREN ALKALIMETALLSALZEN**
PROCESS FOR PRODUCING N-ACYLAMINOCARBOXYLIC ACIDS AND N-ACYLAMINOSULPHONIC ACIDS AND THEIR ALKALINE METAL SALTS
PROCEDE DE PRODUCTION D'ACIDES N-ACYLAMINOCARBOXYLIQUES ET D'ACIDES N-ACYLAMINOSULFONIQUES ET DE LEURS SELS DE METAUX ALCALINS

(30) Priorität: 23.09.1994 DE 4433977
(43) Veröffentlichungstag der Anmeldung: 16.07.1997
(73) Patentinhaber: BASF AKTIENGESELLSCHAFT, 67056 Ludwigshafen (DE)
(72) Erfinder: STRECKER, Beate, D-67059 Ludwigshafen (DE); OFTRING, Alfred, D-67098 Bad Dürkheim (DE); HERTEL, Dieter, D-69181 Leimen (DE); SCHUH, Georg, D-67067 Ludwigshafen (DE)
(86) Internationale Anmeldenummer: EP9503584
(87) Internationale Veröffentlichungsnummer: WO9609278

(56) Entgegenhaltungen:
- EP-A- 0 033 392
- WO-A-95/07881
- DE-A- 1 493 650
- DE-A- 4 408 957
- DE-B- 1 127 359
- US-A- 3 836 551

## Beschreibung

Die vorliegende Erfindung betrifft ein verbessertes Verfahren zur Herstellung von N-Acylaminocarbonsäuren und N-Acylaminosulfonsäuren sowie deren Alkalimetallsalzen aus den technischen Alkalimetallsalzen von Aminocarbonsäuren bzw. Aminosulfonsäuren und Carbonsäurealkylestern.

Aus der DE-OS 20 04 099 ist bekannt, daß man Salze von N-Acylaminocarbonsäuren durch Acylieren der entsprechenden Aminocarbonsäuren bzw. deren Salze mit einer Carbonsäure, einem Carbonsäureester oder einem Carbonsäureamid in Gegenwart einer äquivalenten Menge einer basischen, mit Carbonsäure Salze bildenden Verbindung wie eines Alkali- oder Erdalkalimetallhydroxids bei Temperaturen von 100 bis 250°C, vorzugsweise bei 160 bis 200°C, herstellen kann. Die basische Verbindung dient dabei lediglich der Neutralisation der eingesetzten Aminocarbonsäuren oder Carbonsäuren. Die Umsetzung wird in der Schmelze aus Aminocarbonsäure und Acylierungsmittel oder in Suspension der Aminocarbonsäure in einer Lösung aus einem Acylierungsmittel und einer organischen basischen Stickstoffverbindung, z.B. einem Amin, in einem hochsiedenden organischen Lösungsmittel durchgeführt.

Nachteilig an der geschilderten Reaktionsführung sind jedoch die langen Reaktionszeiten und die niedrigen Ausbeuten, welche im wesentlichen auf die teilweise Zersetzung der Ausgangsverbindungen und der Produkte aufgrund der hohen Reaktionstemperaturen zurückzuführen sind. Das Reaktionsgemisch färbt sich hierbei nämlich leicht dunkel und eine fast immer zu beobachtende schwache Kohlendioxid-Entwicklung kann ebenfalls nur durch Zersetzungsreaktionen erklärt werden.

In der EP-A 033 392 wird die Acylierung von Aminocarbonsäuren oder deren Salzen beschrieben, welche mit Carbonsäureestern und 1,2 bis 2,0 mol Alkalimetallalkoholat gemischt sind. Das Alkalimetallalkoholat dient zur wasserfreien Salzbildung und als Katalysator. Der Einsatz von technischem Alkalimetallsalz wird nicht vorgeschlagen.

In der DE-A 44 08 957 wird ein Herstellungsverfahren für N-Acylaminocarbon- und -sulfonsäuren aus den Alkalimetallsalzen der zugrunde liegenden Aminocarbon- bzw. -sulfonsäuren und Carbonsäurealkylestern beschrieben, bei dem zu einer Suspension der Aminocarbon- bzw. sulfonsäuresalze in den Carbonestern 0,5 bis 30 mol-% starker Basen als Katalysator zugegeben werden. Dieses Verfahren liefert allerdings nur befriedigende Resultate bei Verwendung von reinen Aminocarbon- bzw. -sulfonsäurealkalimetallsalzen mit einem Aktivgehalt von mehr als 95 Gew.-%, bezogen auf den Feststoffgehalt der Alkalimetallsalze.

Verwendet man entsprechende Salze technischer Qualität, treten drastische Selektivitätseinbußen von bis zu 15 % auf.

Aufgabe der vorliegenden Erfindung war es daher, ein effizientes und wirtschaftliches Herstellungsverfahren ausgehend von technischen Aminocarbon- oder -sulfonsäurealkalimetallsalzen bereitzustellen, welches in guten Raum-Zeit-Ausbeuten N-Acylaminocarbonsäuren und N-Acylaminosulfonsäuren bzw. deren Alkalimetallsalze in hohen Reinheiten und Selektivitäten liefert.

Demgemäß wurde ein Verfahren zur Herstellung von N-Acylaminocarbonsäuren und N-Acylaminosulfonsäuren sowie deren Alkalimetallsalzen aus den technischen Alkalimetallsalzen von Aminocarbonsäuren bzw. Aminosulfonsäuren mit einem Aktivgehalt von 50 bis 95 Gew.-%, vorzugsweise 70 bis 94 Gew.-%, insbesondere 80 bis 93 Gew.-%, bezogen auf den Feststoffgehalt der technischen Alkalimetall-Salze, und Carbonsäurealkylestern gefunden, welches dadurch gekennzeichnet ist, daß man
(a) eine Suspension der festen wasserfreien technischen Alkalimetallsalze der Aminocarbonsäuren bzw. Aminosulfonsäuren in den Carbonsäurealkylestern herstellt,
(b) diese Suspension durch Zusatz von mehr als 30 bis 150 mol-% starker Basen zu den Alkalimetallsalzen der N-Acylaminocarbonsäuren bzw. N-Acylaminosulfonsäuren abreagieren läßt und
(c) gewünschtenfalls daraus die freien N-Acylaminocarbonsäuren bzw. N-Acylaminosulfonsäuren in üblicher Weise durch Zugabe von Säuren herstellt.

Bei Schritt (a) geht man beispielsweise so vor, daß man die Carbonsäurealkylester und die festen wasserfreien technischen Alkalimetallsalze der Aminocarbonsäuren bzw. Aminosulfonsäuren in einem geeigneten Gefäß aus Glas oder einem anderen Material vorlegt und mit einem üblichen suspendierenden Rührgerät zu einer feinteiligen Suspension verarbeitet.

Man kann die Suspension in Schritt (a) aber auch dadurch herstellen, daß man die Carbonsäurealkylester und eine wäßrige Lösung der technischen Alkalimetallsalze der Aminocarbonsäuren bzw. Aminosulfonsäuren in einem geeigneten Gefäß aus Glas oder einem anderen Material miteinander vermischt und anschließend unter Erwärmen und Anlegen eines Vakuums das Wasser möglichst rasch aus dem Gemisch entfernt. Überraschenderweise kann beobachtet werden, daß dabei nur in sehr geringem Umfang (meist weniger als zu 2 %) eine Verseifung der Carbonsäurealkylester zu den Alkalimetallsalzen der zugrunde liegenden Carbonsäuren auftritt. Der Vorteil der Verwendung von solchen wäßrigen Lösungen ist, daß man im technischen Syntheseprozeß anfallende wäßrige Lösungen von verunreinigten Alkalimetallsalzen von Aminocarbonsäuren bzw. Aminosulfonsäuren ohne weitere Aufbereitung wie Sprühtrocknung einsetzen kann. Außerdem entfallen das Handling mit Feststoffen betreffende Probleme wie Staubbildung oder gleichmäßige Zudosierung.

Technische Alkalimetallsalze von Aminocarbonsäuren enthalten ein Spektrum von Nebenbestandteilen, im Falle von beispielsweise technischem Sarkosin-Natrium, welches in der Regel durch "Strekker-Reaktion" von Methylamin mit Formaldehyd und Blausäure und anschließende Hydrolyse des gebildeten Methylaminoacetonitril hergestellt wird, sind dies hauptsächlich weitere Aminocarboxylate, z.B. Methyliminodiacetat oder Dimethylglycinat, Carboxylate wie Alkalimetallsalze der Glykolsäure und Formiat.

In der Regel stellt man die Suspension in Schritt (a) aus äquimolaren oder annähernd äquimolaren Mengen von technischen Alkalimetallsalzen von Aminocarbonsäuren bzw. Aminosulfonsäuren und Carbonsäurealkylestern her, wobei ein Überschuß bis zu 15 mol-%, insbesondere bis zu 10 mol-%, einer der beiden Komponenten vertretbar ist. Ein größerer Überschuß an Carbonsäurealkylester, etwa als Verdünnungsmittel, ist normalerweise nicht notwendig.

Zu der gemäß Schritt (a) hergestellten Suspension werden in Schritt (b) die starken Basen hinzugegeben, um die Umsetzung in Gang zu setzen. Der Zusatz der Basen erfolgt meist nach oder während des Aufheizens der Suspension auf Umsetzungstemperatur, kann aber auch schon bei der Herstellung der Suspension in
Schritt (a), beispielsweise zusammen mit den Alkalimetallsalzen der Aminocarbonsäuren bzw. Aminosulfonsäuren, oder kurz vor dem Aufheizen der Suspension erfolgen. Die Basen können als Festsubstanzen oder in gelöster Form, beispielsweise in einem organischen Lösungsmittel wie einem Alkohol, eingesetzt werden.

Die Menge an eingesetzten starken Basen beträgt mehr als 30 bis 150 mol-%, vorzugsweise 70 bis 135 mol-%, insbesondere 80 bis 125 mol-%, vor allem 90 bis 115 mol-%, bezogen auf die technischen Alkalimetallsalze der Aminocarbonsäuren bzw. Aminosulfonsäuren. Man kann eine einzelne Species einer starken Base oder eine Mischung aus verschiedenen Basen verwenden.

Als starke Basen eignen sich insbesondere:
- Alkoholate, vor allem Alkalimetallalkoholate von C₁- bis C₄-Alkanolen, z.B. Natriummethanolat, Natriumethanolat, Natriumisopropylat oder Kalium-tert.-butylat;
- Hydride, z.B. Natriumhydrid, Natriumborhydrid oder Lithiumaluminiumhydrid;
- Alkalimetall- oder Erdalkalimetallhydroxide, z.B. Natriumhydroxid, Kaliumhydroxid, Lithiumhydroxid oder Calciumhydroxid;
- Alkalimetallcarbonate, z.B. Natriumcarbonat, Kaliumcarbonat oder Lithiumcarbonat;
- salzartige Amide, z.B. Lithiumdiisopropylamid;
- Lithiumorganyle wie Alkyllithiumverbindungen, z.B. n-Butyllithium oder Methyllithium, oder Phenyllithium.
Bevorzugt werden hiervon Alkoholate.

Die Umsetzung in Schritt (b) wird in einem relativ schonenden Temperaturbereich vorgenommen, meist bei 50 bis 150°C, insbesondere bei 80 bis 140°C, vor allem bei 100 bis 130°C. Man arbeitet normalerweise bei Normaldruck; eine Umsetzung unter Eigendruck oder erhöhtem Druck ist zwar möglich, bringt aber keine weiteren Vorteile.

Üblicherweise wird nach 1 bis 2 Stunden Umsetzungsdauer nach Basenzugabe im Reaktionsgemisch kein Carbonsäurealkylester mehr durch analytische Methoden, z.B. Infrarot(IR)-Spektroskopie, nachgewiesen. Die bei der Umsetzung aus den Carbonsäurealkylestern entstehenden Alkanole destillieren meist - gegebenenfalls unter vermindertem Druck - aus dem Reaktionsgemisch ab oder verbleiben, zumindest teilweise, im Reaktionsgemisch. Das Reaktionsgemisch liegt nach beendeter Umsetzung in der Regel als viskose Paste vor. Diese kann nach Absenken der Temperatur, etwa auf 80 bis 100°C, durch Zugabe von Wasser gelöst werden. Man erhält so beispielsweise ca. 30 bis 40 gew.-%ige wäßrige Lösungen der Alkalimetallsalze der N-Acylaminocarbonsäuren bzw. N-Acylaminosulfonsäuren.

Sollten die freien N-Acylaminocarbonsäuren oder N-Acylaminosulfonsäuren erhalten werden, stellt man diese gemäß Schritt (c) aus den Alkalimetallsalzen in üblicher Weise durch Zugabe von Säuren her. Als Säuren eignen sich insbesondere Mineralsäuren wie Schwefelsäure, Phosphorsäure oder Salzsäure, welche meist bei Raumtemperatur zu den wäßrigen Lösungen der Alkalimetallsalze der N-Acylaminocarbonsäuren bzw. N-Acylaminosulfonsäuren gegeben werden, so daß sich ein pH-Wert im Bereich von etwa 0 bis 3, insbesondere 1 bis 2, einstellt. Dabei entstehen in der Regel milchige cremige Emulsionen. Vorteilhafterweise werden diese Emulsionen mittels eines üblichen Phasentrennhilfsmittels, z.B. Ketonen wie iso-Butylmethylketon oder Methylethylketon, Alkanolen wie n-Butanol, iso-Butanol oder sek.-Butanol, Ethern wie Methyl-tert.-butylether oder Diisopropylether, Essigsäure-C₁- bis C₄-alkylestern, Propionsäure-C₁-bis C₄-alkylestern oder Acetessigester, welches gleichzeitig mit den Säuren oder nach Bildung der Emulsion zugegeben werden kann, bei leicht erhöhter Temperatur, etwa bei 40 bis 70°C, getrennt. Derartige Phasentrennhilfsmittel sind meist niedrigsiedende, mit Wasser nicht oder wenig mischbare Verbindungen, die kostengünstig großtechnisch einsetzbar sind.

Das erfindungsgemäße Verfahren läßt sich besonders gut anwenden-, wenn man als technische Alkalimetallsalze von Aminocarbonsäuren die Natrium- oder Kaliumsalze von aliphatischen Aminocarbonsäuren mit 2 bis 10 C-Atomen, vorzugsweise 3 bis 6 C-Atomen, insbesondere von Valin, Leucin, Norleucin, Glycin, Alanin, β-Alanin, ε-Aminocapronsäure, α-Aminoisobuttersäure, Sarkosin (N-Methylglycin), Asparaginsäure, Glutaminsäure oder Iminodiessigsäure einsetzt. Es lassen sich aber auch die Natrium- oder Kaliumsalze von anderen natürlichen α-Aminosäuren, von Oligopeptiden oder von aromatischen oder cycloaliphatischen Aminocarbonsäuren, z.B. Anthranilsäure, Phenylglycin, Phenylalanin oder 1-Aminocyclohexan-1-carbonsäure, verwenden. Unter Aminocarbonsäuren sind hier vor allem Verbindungen mit einer primären oder sekundären Aminogruppe und einer oder zwei Carboxylgruppen pro Molekül zu verstehen; es können prinzipiell jedoch auch Verbindungen mit mehr als einer Aminogruppe und/oder mehr als zwei Carboxylgruppen eingesetzt werden, die Menge an Carbonsäurealkylestern richtet sich dann nach der Anzahl der Aminogruppen. Alle Carboxylgruppen liegen praktisch vollständig in der Salzform vor.

Das erfindungsgemäße Verfahren läßt sich ebenfalls besonders gut anwenden, wenn man als technische Alkalimetallsalze von Aminosulfonsäuren die Natrium- oder Kaliumsalze von aliphatischen Aminosulfonsäuren mit 2 bis 10 C-Atomen, vorzugsweise 2 bis 4 C-Atomen, einsetzt. Insbesondere sind hier die entsprechenden Salze von Taurin (2-Aminoethansulfonsäure) und N-Methyltaurin von Interesse. Genau wie bei den Aminocarbonsäuren können die verwendeten Aminosulfonsäuren, welche ebenfalls praktisch vollständig in der Alkalimetallsalz-Form vorliegen, mehrere Aminogruppen und/ oder Sulfonsäuregruppen aufweisen.

Neben den Natrium- oder Kaliumsalzen lassen sich ebenfalls die entsprechenden Lithiumsalze einsetzen.

Als Carbonsäurealkylester kommen vor allem Fettsäureniedrigalkylester, d.h. C₁- bis C₄-Alkylester von gesättigten oder ungesättigten C₆- bis C₃₀-Monocarbonsäuren, in Betracht. Insbesondere eignen sich die Methylester von gesättigten oder ungesättigten C₈- bis C₂₀-Monocarbonsäuren, z.B. Laurinsäuremethylester, Myristinsäuremethylester, Palmitinsäuremethylester, Stearinmethylester, Ölsäuremethylester, Linolsäuremethylester oder Linolensäuremethylester oder Gemische solcher Ester von natürlich vorkommenden langkettigen Fettsäuren, z.B. Kokosfettsäuremethylester, Palmölfettsäuremethylester, Palmkernölfettsäuremethylester, Talgfettsäuremethylester, Sojaölfettsäuremethylester, Sonnenblumenölfettsäuremethylester oder Rapssamenölfettsäuremethylester, sowie entsprechende Ester synthetisch erzeugter C₈- bis C₂₀-Monocarbonsäuren oder deren Gemische. Das erfindungsgemäße Verfahren läßt sich sowohl in diskontinuierlicher als auch in kontinuierlicher Fahrweise durchführen.

Mit Hilfe des erfindungsgemäßen Verfahrens lassen sich reine N-Acylaminocarbonsäuren und N-Acylaminosulfonsäuren sowie deren Alkalimetallsalze oder Gemische solcher Verbindungen mit unterschiedlichen Acylresten und/oder unterschiedlichen Aminocarbonsäure- bzw. Aminosulfonsäure-Grundkörpern herstellen. Derartige Verbindungen eignen sich bekanntermaßen als Emulgatoren oder Tenside auf verschiedensten technischen Gebieten.

Das erfindungsgemäße Verfahren liefert in guten Raum-Zeit-Ausbeuten die gewünschten Produkte in hoher Reinheit. Durch die relativ schonende Temperaturführung treten keine Zersetzungsreaktionen auf, die zu Ausbeuteminderungen und Dunkelfärbungen der Produkte führen. Die Umsetzungen sind in wesentlich kürzerer Zeit vollständig abgelaufen.

Durch den Einsatz von Carbonsäurealkylestern als kostengünstigen und leicht handhabbaren Acylierungskomponenten und die Vermeidung von Salzanfall bei der Acylierung ist das erfindungsgemäße Verfahren, insbesondere in einer kontinuierlichen Fahrweise, großtechnisch höchst attraktiv.

Bemerkenswert ist beim vorliegenden erfindungsgemäßen Verfahren, daß durch Erhöhung des Anteils an starken Basen im Reaktionsgemisch über die üblichen katalytischen Mengen hinaus der Verlust an Selektivität der Reaktion bei Verwendung von technischen, d.h. verunreinigten Aminocarbon- bzw. -sulfonsäurealkalimetallsalzen anstelle der reinen Salze im wesentlichen kompensiert wird. Dies ist um so überraschender, als in der Literatur das Vorurteil besteht, daß aliphatische Carbonsäureester mit sekundären Aminen in Gegenwart von äquimolaren Mengen Base wie Natriummethanolat nicht zu den entsprechenden Amiden reagieren (R.J. de Feoand und P.D. Strickler, J. Org. Chem. 28, 2915-2917, 1963). Das nachfolgende Beispiel 2 mit Sarkosin-Natrium, einem sekundären Amin, wiederlegt klar dieses Vorurteil.

### Beispiele

Beispiel 1 (zum Vergleich): Herstellung von N-Oleoylsarkosin mit üblichen katalytischen Mengen Natriummethanolat
1 Mol Ölsäuremethylester (Edenor®ME TiO5 der Firma Henkel) und 1 Mol wasserfreies technisches Sarkosin-Natrium-Pulver (Aktivgehalt: 84,4 Gew.-% Sarkosin-Natrium) wurden in ein Becherglas gefüllt und mit einem "Ultra-Turrax" zu einer feinen Suspension vermischt. Die Suspension wurde in einen Glasreaktor übergeführt und auf 120°C erwärmt. Unter einer Stickstoffatomosphäre wurden innerhalb von 10 Minuten 25 mol-% Natriummethanolat als ca. 30 gew.-%ige Lösung in Methanol unter Rühren zudosiert. Nach beendeter Zugabe wurde bei 120°C so lange nachgerührt, bis im IR-Spektrum des Reaktionsgemisches kein Carbonester mehr nachgewiesen werden konnte (ca. 3,5 Stunden). Anschließend wurde das Reaktionsgemisch auf 100°C abgekühlt und mit 600 ml Wasser versetzt. Es wurde so lange gerührt, bis eine klare wäßrige Lösung entstanden war.

Zu dieser Lösung wurden 100 g 96 gew.-%ige Schwefelsäure zugesetzt. Es bildete sich eine cremige Emulsion, die sich nach Zusatz von 80 g Methylethylketon zu einem Zweiphasengemisch auftrennte. Dieses wurde im Scheidetrichter getrennt, die wäßrige Phase wurde verworfen. Von der organischen Phase wurde das Methylethylketon bei < 50°C und ca. 40 mbar abdestilliert und wiederverwendet. Es verblieb ein orangebraunes Öl mit einem mittels HPLC bestimmten Gehalt von 76,7 Gew.-% N-Acylsarkosin; die Ausbeute betrug 290 g (entsprechend 83,5 % der Theorie).

Beispiel 2 (erfindungsgemäß): Herstellung von N-Oleoylsarkosin mit äquimolaren Mengen Natriummethanolat

Der unter Beispiel 1 beschriebene Versuch wurde wiederholt mit dem Unterschied, daß 100 mol-% anstelle von 25 mol-% Natriummethanolat eingesetzt wurden. Nach Aufarbeitung erhielt man ein orangebraunes Öl mit einem mittels HPLC bestimmten Gehalt von 82,9 Gew.-% N-Acylsarkosin; die Ausbeute betrug 322 g (entsprechend 92,5 % der Theorie).

Beispiel 3 (erfindungsgemäß): Herstellung von N-Oleoylsarkosin mit überstöchiometrischen Mengen Natriummethanolat

Der unter Beispiel 1 beschriebene Versuch wurde wiederholt mit dem Unterschied, daß 110 mol-% anstelle von 25 mol-% Natriummethanolat eingesetzt wurden. Nach Aufarbeitung erhielt man ein orangebraunes Öl mit einem mittels HPLC bestimmten Gehalt von 85,2 Gew.-% N-Acylsarkosin; die Ausbeute betrug 320 g (entsprechend 91,9 % der Theorie).

## Patentansprüche

1. Verfahren zur Herstellung von N-Acylaminocarbonsäuren und N-Acylaminosulfonsäuren sowie deren Alkalimetallsalzen aus den technischen Alkalimetallsalzen von Aminocarbonsäuren bzw. Aminosulfonsäuren mit einem Aktivgehalt von 50 bis 95 Gew.-%, bezogen auf den Feststoffgehalt der technischen Alkalimetallsalze, und Carbonsäurealkylestern, dadurch gekennzeichnet, daß man
(a) eine Suspension der festen wasserfreien technischen Alkalimetallsalze der Aminocarbonsäuren bzw. Aminosulfonsäuren in den Carbonsäurealkylestern herstellt,
(b) diese Suspension durch Zusatz von mehr als 30 bis 150 mol-% starker Basen zu den Alkalimetallsalzen der N-Acylaminocarbonsäuren bzw. N-Acylaminosulfonsäuren abreagieren läßt und
(c) gewünschtenfalls daraus die freien N-Acylaminocarbonsäuren bzw. N-Acylaminosulfonsäuren in üblicher Weise durch Zugabe von Säuren herstellt.

2. Verfahren nach Anspruch 1, dadurch gekennzeichnet, daß man in Schritt (a) eine Suspension aus äquimolaren oder annähernd äquimolaren Mengen von technischen Alkalimetallsalzen von Aminocarbonsäuren bzw. Aminosulfsäuren und Carbonsäurealkylestern herstellt.

3. Verfahren nach Anspruch 1 oder 2, dadurch gekennzeichnet, daß man in Schritt (b) die starken Basen in Mengen von 70 bis 120 mol-%, bezogen auf die technischen Alkalimetallsalze der Aminocarbonsäuren bzw. Aminosulfonsäuren, einsetzt.

4. Verfahren nach den Ansprüchen 1 bis 3, dadurch gekennzeichnet, daß man in Schritt (b) Alkalimetallalkoholate als starke Basen einsetzt.

5. Verfahren nach den Ansprüchen 1 bis 3, dadurch gekennzeichnet, daß man die Umsetzung des Schrittes (b) bei Temperaturen von 50 bis 150°C vornimmt.

6. Verfahren nach den Ansprüchen 1 bis 5, wobei man als technische Alkalimetallsalze von Aminocarbonsäuren die
Natrium- oder Kaliumsalze von aliphatischen Aminocarbonsäuren mit 2 bis 10 C-Atomen einsetzt.

7. Verfahren nach den Ansprüchen 1 bis 5, wobei man als technische Alkalimetallsalze von Aminosulfonsäuren die
Natrium- oder Kaliumsalze von aliphatischen Aminosulfonsäuren mit 2 bis 10 C-Atomen einsetzt.

8. Verfahren nach den Ansprüchen 1 bis 7, wobei man als Carbonsäurealkylester die C₁- bis C₄-Alkylester von gesättigten oder ungesättigten C₆- bis C₃₀-Monocarbonsäuren einsetzt.

## Claims

1. A process for preparing N-acylamino carboxylic acids and N-acylamino sulfonic acids and their alkali metal salts from the technical alkali metal salts of amino carboxylic acids and amino sulfonic acids, respectively, with an active content of 50-95% by weight, based on the solids content of the technical alkali metal salts, and from alkyl carboxylates, which comprises
(a) preparing a suspension of the solid anhydrous technical alkali metal salts of the amino carboxylic acids or amino sulfonic acids in the alkyl carboxylates,
(b) reacting this suspension by adding more than 30 to 150 mol% of strong bases to give the alkali metal salts of the N-acylamino carboxylic acids or N-acylamino sulfonic acids, and
(c) if required preparing therefrom the free N-acylamino carboxylic acids or N-acylamino sulfonic acids in a conventional way by adding acids.

2. A process as claimed in claim 1, wherein in step (a) a suspension of equimolar or approximately equimolar amounts of technical alkali metal salts of amino carboxylic acids or amino sulfonic acids and alkyl carboxylates is prepared.

3. A process as claimed in claim 1 or 2, wherein in step (b) the strong bases are used in amounts of from 70 to 120 mol%, based on the technical alkali metal salts of the amino carboxylic acids or amino sulfonic acids.

4. A process as claimed in any of claims 1 to 3, wherein in step (b) alkali metal alcoholates are used as strong bases.

5. A process as claimed in any of claims 1 to 3, wherein the reaction in step (b) is carried out at from 50 to 150° C.

6. A process as claimed in any of claims 1 to 5, wherein the sodium or potassium salts of aliphatic amino carboxylic acids having 2 to 10 carbons are used as technical alkali metal salts of amino carboxylic acids.

7. A process as claimed in any of claims 1 to 5, wherein the sodium or potassium salts of aliphatic amino sulfonic acids having 2 to 10 carbons are used as technical alkali metal salts of amino sulfonic acids.

8. A process as claimed in any of claims 1 to 7, wherein the C₁-C₄-alkyl esters of saturated or unsaturated C₆-C₃₀-monocarboxylic acids are used as alkyl carboxylates.

## Revendications

1. Procédé de préparation d'acides N-acylaminocarboxyliques et d'acides N-acylaminosulfoniques ainsi que de leurs sels de métal alcalin à partir de sels de métal alcalin de qualité technique d'acides aminocarboxyliques ou bien d'acides aminosulfoniques ayant une teneur en matière active de 50 à 95% en poids, par rapport à la teneur en matière solide des sels de métal alcalin de qualité technique et d'esters alkyliques d'acides carboxyliques, caractérisé en ce que
(a) on prépare une suspension des sels de métal alcalin solides exempts d'eau de qualité technique des acides aminocarboxyliques ou bien des acides aminosulfoniques dans les esters alkyliques d'acides carboxyliques,
(b) on fait réagir cette suspension en ajoutant de plus de 30 à 150% en moles de bases fortes aux sels de métal alcalin des acides N-acylaminocarboxyliques ou bien des acides N-acylaminosulfoniques et
(c) on y prépare éventuellement des acides N-acylaminocarboxyliques ou bien des acides N-acylaminosulfoniques libres d'une manière habituelle en ajoutant des acides.

2. Procédé selon la revendication 1, caractérisé en ce que dans l'étape (a), on prépare une suspension de quantité équimolaire ou approximativement équimolaire de sels de métal alcalin de qualité technique d'acides aminocarboxyliques ou bien d'acides aminosulfoniques et d'esters alkyliques d'acides carboxyliques.

3. Procédé selon la revendication 1 ou 2, caractérisé en ce que dans l'étape (b), on met en oeuvre les bases fortes en des quantités de 70 à 120% en moles, par rapport aux sels de métal alcalin de qualité technique des acides aminocarboxyliques ou bien des acides aminosulfoniques.

4. Procédé selon les revendications 1 à 3, caractérisé en ce que dans l'étape (b), on met en oeuvre des alcoolates de métal alcalin en tant que bases fortes.

5. Procédé selon les revendications 1 à 3, caractérisé en ce qu'on réalise la réaction de l'étape (b) à des températures de 50 à 150°C.

6. Procédé selon les revendications 1 à 5, dans lequel on met en oeuvre en tant que sels de métal alcalin de qualité technique d'acides aminocarboxyliques, les sels de sodium ou de potassium d'acides aminocarboxyliques aliphatiques ayant 2 à 10 atomes de carbone.

7. Procédé selon les revendications 1 à 5, dans lequel on met en oeuvre en tant que sels de métal alcalin de qualité technique d'acides aminosulfoniques, les sels de sodium ou de potassium d'acides aminosulfoniques aliphatiques ayant 2 à 10 atomes de carbone

8. Procédé selon les revendications 1 à 7, dans lequel on met en oeuvre en tant qu'esters alkyliques d'acides carboxyliques, les esters alkyliques en C₁ à C₄ provenant d'acides monocarboxyliques en C₆ à C₃₀ insaturés ou saturés.
